(19) Europäisches Patentamt
European Patent Office
Office européen des brevets

(11) Veröffentlichungsnummer : **0 115 298**
**B1**

(12) # EUROPÄISCHE PATENTSCHRIFT

(45) Veröffentlichungstag der Patentschrift : **17.08.88**

(51) Int. Cl.⁴ : **C 08 G 18/79**, C 08 G 18/76

(21) Anmeldenummer : **84100523.4**

(22) Anmeldetag : **19.01.84**

(54) **Verfahren zur Herstellung von Isocyanuratgruppen aufweisenden Polyisocyanaten, die nach diesem Verfahren erhältlichen Verbindungen und ihre Verwendung zur Herstellung von Polyurethanen.**

(30) Priorität : **01.02.83 DE 3303221**

(43) Veröffentlichungstag der Anmeldung : **08.08.84 Patentblatt 84/32**

(45) Bekanntmachung des Hinweises auf die Patenterteilung : **17.08.88 Patentblatt 88/33**

(84) Benannte Vertragsstaaten : **DE FR GB IT NL**

(56) Entgegenhaltungen :
**DE-A- 2 525 017**
**US-A- 3 108 092**

(73) Patentinhaber : **BAYER AG**
**Konzernverwaltung RP Patentabteilung**
**D-5090 Leverkusen 1 Bayerwerk (DE)**

(72) Erfinder : **Scholl, Hans-Joachim, Dr.**
**Rudolf-Sohm-Strasse 28**
**D-5000 Koeln 60 (DE)**
Erfinder : **Pedain, Josef, Dr.**
**Haferkamp 6**
**D-5000 Koeln 80 (DE)**
Erfinder : **Riberi, Bernd, Dr.**
**Jakob-Boehme-Strasse 2**
**D-5000 Koeln 80 (DE)**
Erfinder : **Mennicken, Gerhard, Dr.**
**Am Wasserturm 16**
**D-5090 Leverkusen 3 (DE)**

**EP 0 115 298 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein neues Verfahren zur Herstellung von Isocyanuratgruppen aufweisenden Polyisocyanaten durch teilweise Trimerisierung der Isocyanat-Gruppen von organischen Polyisocyanaten unter (Mit)verwendung von bestimmten, nachstehend näher beschriebenen, alkylsubstituierten Phenylendiisocyanaten als Ausgangspolyisocyanat, die nach diesem Verfahren erhältlichen Verbindungen, sowie ihre Verwendung zur Herstellung von Polyurethanen nach dem Isocyanat-Polyadditionsverfahren.

Verfahren zur Herstellung von Isocyanuratgruppen aufweisenden Polyisocyanaten sind in großer Zahl bekannt geworden (J.H. Saunders und K.C. Frisch, Polyurethanes Chemistry and Technology, Part I, S. 94 ff [1962]). Bei diesen Verfahren werden Trimerisierungskatalysatoren der unterschiedlichsten, in der genannten Literaturstelle beispielhaft genannten Art verwendet. Neben den in der genannten Literaturstelle beispielhaft offenbarten Trimerisierungskatalysatoren haben sich in neuerer Zeit insbesondere Mannich-Basen (DE-A-2 551 634 oder DE-A-2 641 380) zur Trimerisierung von aromatischen Isocyanaten, quaternäre Hydroxyalkylammoniumhydroxide (EP-B-37 65, US-A-4 288 586, EP-B-10 589 oder US-A-4 324 879) zur Trimerisierung aliphatischer oder cycloaliphatischer Polyisocyanate oder komplexierte basische Natrium- oder Kaliumverbindungen (DE-A-31 00 262 oder DE-A-31 00 263) als universell einsetzbare Trimerisierungskatalysatoren bewährt. Auch die in DE-A-2 616 415 und DE-A-2 616 416 beschriebenen Verfahren und Katalysatoren sind gut geeignet.

Ein wichtiges Einsatzgebiet für die bislang bekannt gewordenen, Isocyanuratgruppen aufweisenden Polyisocyanate stellt ihre Verwendung als Reaktionskomponente in 2-Komponenten-Polyurethanlacken in Kombination mit Hydroxylgruppen aufweisenden Reaktionspartnern, beispielsweise in Kombination mit Polyhydroxy-Polyacrylaten, Polyhydroxyl-Polyestern oder Hydroxylgruppen enthaltenden Alkydharzen dar.

Den meisten bekannten, Isocyanuratgruppen aufweisenden Polyisocyanaten haften indessen noch eine Reihe verbesserungswürdiger Nachteile an. So sind die Isocyanuratgruppen aufweisende Polyisocyanate des Standes der Technik mit unpolaren Lacklösungsmitteln wie z. B. aliphatischen oder aromatischen Kohlenwasserstoffen nur schlecht verdünnbar. Dieser Nachteil äußert sich darin, daß beim Vermischen der Isocyanuratgruppen enthaltenden Polyisocyanate mit aliphatischen oder aromatischen Kohlenwasserstoffen bzw. mit Polyestern Polyacrylaten oder Alkydharzen, die in diesen Lösemitteln gelöst sind, Trübungen und Ausfällungen auftreten, die die Herstellung von Lacken unmöglich machen.

Ein weiterer Nachteil der Isocyanuratgruppen aufweisenden Polyisocyanate des Standes der Technik is auf deren oft anzutreffende Unverträglichkeit mit anderen Bindemitteln und Reaktionspartnern zurückzuführen, die sich in einer oft zu kurzen Verarbeitungszeit der gebrauchsfertigen Lackmischungen äußert. Der Grund für diese oft zu kurze Verarbeitungszeit ist insbesondere darauf zurückzuführen, daß bei Ausbildung von nur wenigen Urethangruppen in den gebrauchsfertigen Lacken die ohnehin geringe Löslichkeit bzw. Verträglichkeit noch schlechter wird und das Bindemittel aus der Lacklösung ausfällt. Der naheliegende Gedanke, die schlechte Verträglichkeit durch Mitverwendung von polaren Lösungsmitteln zu beseitigen, führt zu einer beträchtlichen Verteuerung der Lacke. Die in der DE-B-2 414 413 empfohlene Modifizierung der Polyisocyanate mit Fettalkoholen beinhaltet eine wesentliche Verbesserung, ist jedoch mit dem Nachteil behaftet, daß durch diese Modifizierung Isocyanatgruppen verbraucht werden, die nicht mehr zur Ausbildung von Vernetzungsstellen zur Verfügung stehen und die Funktionalität der Polyisocyanate vermindern.

Es war daher die Aufgabe der vorliegenden Erfindung, neue, auf einfachem Wege zugängliche Isocyanuratgruppen aufweisende Polyisocyanate zur Verfügung zu stellen, die mit wenig polaren Löse- und Verdünnungsmitteln verträglich und mit Hydroxyl-Polyestern, -Polyacrylaten und -Alkydharzen ohne Eintrübung auch in Gegenwart von unpolaren Lösemitteln mischbar sind und im Gemisch mit diesen Reaktionspartnern eine verbesserte, möglichst lange Verarbeitungszeit aufweisen.

Diese Aufgabe konnte mit dem nachstehend näher beschriebenen erfindungsgemäßen Verfahren gelöst werden.

Gegenstand der Erfindung ist ein Verfahren zur Herstellung von Isocyanurat-Gruppen aufweisenden Polyisocyanaten durch Trimerisierung eines Teils der Isocyanatgruppen von organischen Polyisocyanaten in Gegenwart von Trimerisierungskatalysatoren und Abbruch der Trimerisierungsreaktion beim jeweils gewünschten Trimerisierungsgrad durch Zugabe eines Katalysatorengifts und/oder durch thermische Zersetzung des eingesetzten Trimerisierungskatalysators, dadurch gekennzeichnet, daß man als zu trimerisierende Polyisocyanate :

a) Verbindungen oder Gemische von Verbindungen der Formel

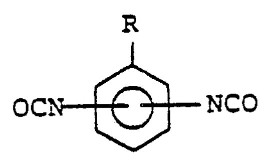

in welcher R für einen gesättigten, aliphatischen Kohlenwasserstoffrest mit 8 bis 15 Kohlenstoffatomen

steht, und gegebenenfalls ;

b) bis zu 95 NCO-Äquivalent-%, bezogen auf die Summe a) + b), an anderen organischen Polyisocyanaten

verwendet.

Gegenstand der Erfindung sind auch die nach diesem Verfahren erhältlichen, Isocyanurat-Gruppen aufweisenden Polyisocyanate.

Gegenstand der Erfindung ist auch die Verwendung der nach diesem Verfahren erhältlichen, Isocyanuratgruppen aufweisenden Polyisocyanate, gegebenenfalls in von überschüssigen Ausgangspolyisocyanaten befreiter Form und/oder gegebenenfalls in mit Blockierungsmittel für Isocyanat-Gruppen blockierter Form als Isocyanatkomponente zur Herstellung von Polyurethanen nach dem Isocyanat-Polyadditions-Verfahren.

Beim erfindungsgemäßen Verfahren werden als erfindungswesentliche Ausgangskomponenten a) Diisocyanate der bereits genannten allgemeinen Formel eingesetzt, für welche R die bereits oben genannte Bedeutung hat. Vorzugsweise werden solche Diisocyanate dieser Art eingesetzt, die als Homologen- und Isomerengemisch vorliegen, und in welchen R für einen gesättigten, geradkettigen, aliphatischen Kohlenwasserstoffrest mit 8 bis 15, insbesondere 10 bis 13 Kohlenstoffatomen steht. Die Herstellung dieser bevorzugt, bzw. besonders bevorzugt als erfindungswesentliche Ausgangskomponente a) einzusetzenden Diisocyanate ist beispielsweise in der EP-A-58 368 bzw. der DE-A-31 05 776 beschrieben, wobei die in EP-A-58 368 beschriebenen Diisocyanate erfindungsgemäß besonders bevorzugt sind. Außer diesen bevorzugten bzw. besonders bevorzugten Ausgangsmaterialien a) können beim erfindungsgemäßen Verfahren als weitere Ausgangskomponenten a) auch solche Diisocyanate der genannten allgemeinen Formel eingesetzt werden, in welchen R für einen verzweigten aliphatischen Kohlenwasserstoffrest mit 8 bis 15 Kohlenstoffatomen steht, und wie sie beispielsweise gemäß US-A-2 986 576 zugänglich sind.

Die erfindungswesentliche Komponente a) wird beim erfindungsgemäßen Verfahren entweder als alleinige Ausgangskomponente oder zusammen mit bis zu 95 NCO-Äquivalent-%, bezogen auf alle Isocyanatgruppen der Ausgangspolyisocyanate, an anderen Polyisocyanaten b) eingesetzt.

Bei diesen gegebenenfalls mitzuverwendenden Polyisocyanaten b) handelt es sich beispielsweise um :

b1) aromatische Polyisocyanate eines über 173, vorzugsweise zwischen 174 und 250 liegenden Molekulargewichts, wie z. B. 2,4- oder 2,6-Toluylendiisocyanat, 2,4'-Diphenylmethandiisocyanat, 4,4'-Diphenylmethandiisocyanat, Naphthylen-1,5-diisocyanat, 4,4',4''-Triisocyanatotriphenyl-methan, 2,4,6-Triisocyanatotoluol oder Polyphenyl-polymethylen-polyisocyanate, wie sie durch Anilin-Formaldehyd-Kondensation und anschließende Phosgenierung hergestellt werden (« Roh-MDI ») ;

b2) aliphatische oder cycloaliphatische Polyisocyanate eines über 139, vorzugsweise zwischen 140 und 250 liegenden Molekulargewichts, wie z. B. Tetramethylendiisocyanat, Hexamethylendiisocyanat, Dodecamethylendiisocyanat, Cyclobutan-1,3-diisocyanat, Cyclohexan-1,3- und -1,4-diisocyanat, 1-Isocyanato-3,3,5-trimethyl-5-isocyanatomethyl-cyclohexan (IPDI), Perhydro-2,4- und/oder -2,6-diisocyanatotoluol oder Perhydro-2,4'- und/oder -4,4'-diisocyanatodiphenylmethan ; oder um :

b3) einen NCO-Gehalt von ca. 1 bis ca. 11 Gew.-% aufweisende NCO-Präpolymere auf Basis von von (i) den erfindungswesentlichen Diisocyanaten a) und/oder den unter b1) und b2) genannten Polyisocyanaten und (ii) den aus der Polyurethanchemie insbesondere für 2-Komponenten-Polyurethanlacke bekannten Polyhydroxylverbindungen des Molekulargewichtsbereiches 62 bis 5 000, vorzugsweise 200 bis 2 000, der in US-A-4 289 813, Kolonne 3, Zeile 47 — Kolonne 4, Zeile 12 beispielhaft offenbarten Art.

Beliebige Gemische der unter b1) bis b3) beispielhaft genannten Polyisocyanate können ebenfalls beim erfindungsgemäßen Verfahren als Komponente b) eingesetzt werden. Vorzugsweise werden die unter b1) beispielhaft genannten aromatischen Diisocyanate verwendet, z. B. 2,4-und/oder 2,6-Diisocyanatotoluol, 2,4-Diisocyanatotoluol bzw. dessen technischen Gemische mit bis zu 65 Gew.-% an 2,6-Diisocyanatotoluol, bezogen auf Gesamtmenge der Diisocyanatotoluol-Isomeren, stellen die besonders bevorzugte Komponente b) dar.

Gemäß einer bevorzugten Ausführungsform des erfindungsgemäßen Verfahrens kommen, bezogen auf alle Isocyanatgruppen der Ausgangspolyisocyanate, 5 bis 70 Äquivalent-% der Ausgangskomponente a), insbesondere der als bevorzugt gekennzeichneten Ausgangskomponente b), insbesondere der besonders bevorzugten Komponente b), zum Einsatz.

Als Trimerisierungskatalysatoren kommen alle bekannten Trimerisierungskatalysatoren des Standes der Technik in Betracht, wie sie beispielsweise in den oben genannten Veröffentlichungen beispielhaft genannt sind. Besonders bevorzugte Trimerisierungskatalysatoren sind die beispielsweise in DE-A-2 551 634 beschriebenen Mannich-Basen, die beispielsweise in US-A-4 324 879 oder US-A-4 288 586 beschriebenen quaternären Hydroxyalkylammoniumhydroxide, die beispielsweise in DE-A-3 100 262 bzw. DE-A-3 100 263 beschriebenen, komplexierten basischen Natrium- oder Kaliumverbindungen, oder die beispielsweise in DE-A-2 616 415 und DE-A-2 616 416 beschriebenen Triazinsalze.

Die Beendigung der erfindungsgemäßen Trimerisierungsreaktionen erfolgt entweder, bei Verwendung von thermisch labilen Trimerisierungskatalysatoren, thermisch oder vorzugsweise durch Zugabe von Katalysatorengiften der beispielsweise in den zuletzt genannten Veröffentlichungen genannten Art.

Das erfindungsgemäße Verfahren kann sowohl lösungsmittelfrei, als auch in Anwesenheit von

inerten Lösungs- und Verdünnungsmitteln durchgeführt werden. Als inerte Lösungsmittel können unpolare Verdünnungsmittel wie Toluol, Xylol, höhere Aromaten, Leichtbenzin, Testbenzin und $C_{12}$-$C_{20}$-Alkylsulfonsäureester, aber auch inerte polare Lösungsmittel wie Ester und Ketone bzw. Gemische derartiger Lösungsmittel eingesetzt werden.

Bevorzugt setzt man Gemische von aromatischen Kohlenwasserstoffen und Estern ein, z. B. ein Gemisch von Butylacetat und Xylol.

Die erfindungsgemäße Trimerisierungsreaktion wird im allgemeinen im Temperaturbereich zwischen 10 bis 100 °C, vorzugsweise 20 bis 80 °C durchgeführt. Die optimale Reaktionstemperatur richtet sich nach der Art der eingesetzten Ausgangspolyisocyanate und der Art der eingesetzten Trimerisierungskatalysatoren und kann in einem einfachen Vorversuch ermittelt werden.

Die erfindungsgemäße Trimerisierungsreaktion wird im allgemeinen bei Erreichen eines Trimerisierungsgrads (Trimerisierungsgrad = Prozentsatz der trimerisierten Isocyanatgruppen, bezogen auf Gesamtmenge der im Ausgangspolyisocyanat vorliegenden Isocyanatgruppen) von 10 bis 70 % abgebrochen. Der Verlauf der Reaktion kann z. B. durch fortlaufende Bestimmung des Brechungsindex' verfolgt werden.

Bei der lösungsmittelfreien Durchführung des erfindungsgemäßen Verfahrens, gegebenenfalls unter anschließender Entfernung von überschüssigem Ausgangsisocyanat, beispielsweise im Dünnschichtverdampfer, liegt der Trimerisierungsgrad im allgemeinen zwischen 10 und 40 %. Bei der Durchführung des erfindungsgemäßen Verfahrens in Gegenwart von Lösungsmitteln ohne anschließende Entfernung von nicht-umgesetztem Ausgangsisocyanat liegt der Trimerisierungsgrad im allgemeinen zwischen 50 und 70 %.

Dabei ist es ein besonderer Vorteil des erfindungsgemäßen Verfahrens, daß bei einem Trimerisierungsgrad von ca. 50 bis 70 % der Gehalt an monomeren Isocyanaten überrraschenderweise sehr niedrig ist und eine anschließende Entfernung von monomeren Isocyanaten durch Extraktion oder Destillation nicht notwendig ist.

Der Abbruch der Trimerisierungsreaktion erfolgt, wie bereits dargelegt, durch thermische oder chemische Desaktivierung des Katalysators.

Vorzugsweise werden beim erfindungsgemäßen Verfahren im Falle der Mitverwendung von Ausgangskomponenten b) vorab hergestellte Gemische der Komponenten a) und b) eingesetzt. Gemäß einer besonderen Ausführungsform des erfindungsgemäßen Verfahren ist es jedoch auch möglich, zunächst in einem ersten Reaktionsschritt einen Teil, d. h. bis maximal 30 %, vorzugsweise bis maximal 5 %, der Isocyanatgruppen der Komponente a) oder der Komponente b) zu trimerisieren, anschließend die nichttrimerisierte Komponente b) oder a) dem Reaktionsgemisch hinzuzufügen und die Trimerisierungsreaktion zum Abschluß zu bringen.

In einem solchem Falle muß jedoch darauf geachtet werden, daß die Beendigung der Trimerisierungsreaktion frühestens dann erfolgt, wenn nach erfolgter Zugabe der nicht-trimerisierten Ausgangskomponente weitere 5 %, vorzugsweise weitere 10 % aller ursprünglich in den Ausgangspolyisocyanaten vorliegender Isocyanatgruppen trimerisiert sind. So ist es beispielsweise möglich, von 100 NCO-Äquivalenten der Komponente a) oder b) max. 30 NCO-Äquivalente zu trimerisieren, dem Reaktionsgemisch dann 100 NCO-Äquivalente der Komponente b) oder a) einzuverleiben, die Trimerisierungsreaktion fortzusetzen und im Falle der Trimerisierung von 30 NCO-Äquivalenten der ersten Ausgangskomponente frühestens nach der Trimerisierung von insgesamt 20, vorzugsweise von insgesamt 25 NCO-Äquivalenten abzubrechen.

Die erfindungsgemäßen Verfahrensprodukte können (insbesondere im Falle der lösungsmittelfreien Durchführung des erfindungsgemäßen Verfahrens) in an sich bekannter Weise, beispielweise durch Dünnschichtdestillation oder Extraktion, von überschüssigen, nicht-umgesetzten Ausgangsisocyanaten befreit werden, so daß Isocyanuratgruppen aufweisende Polyisocyanate mit einem Gehalt an monomeren Ausgangsisocyanaten unter 3 Gew.-%, vorzugsweise unter 0,7 Gew.-%, erhältlich sind.

Die Entfernung von überschüssigen Ausgangsisocyanaten erfolgt vorzugsweise, wenn eine Anwendung der Verfahrensprodukte für Polyurethanlacke vorgesehen ist. Vor ihrer Verwendung als Isocyanat-Komponente in Zweikomponenten-Polyurethanlacken können die erfindungsgemäßen Verfahrensprodukte noch modifiziert werden, beispielsweise durch Einführung von Urethan-, Harnstoff-, Biuret- oder Allophanatgruppen.

Selbstverständlich ist eine Verwendung der erfindungsgemäßen Verfahrensprodukte auch ohne Entfernung von überschüssigen Ausgangsisocyanaten möglich, beispielsweise zur Herstellung von Polyurethanschaumstoffen.

Die erfindungsgemäßen Verfahrensprodukte können selbstverständlich in an sich bekannter Weise mit geeigneten Blockierungsmitteln für Isocyanatgruppen wie z. B. Phenol, ε-Caprolactam, Malonsäurediethylester oder Acetessigsäureethylester blockiert werden.

Die erfindungsgemäßen Verfahrensprodukte bzw. ihre durch die genannte Blockierungsreaktion erhaltenen Derivate stellen wertvolle Ausgangsmaterialien zur Herstellung von Polyurethankunststoffen nach dem Isocyanat-Polyadditionsverfahren dar. Sie eignen sich insbesondere als Isocyanatkomponente in Zweikomponentenpolyurethanlacken.

Die folgenden Beispiele erläutern die Erfindung. Alle Prozentangaben betreffen, soweit nicht anderslautend ausgeführt, Gewichtsprozente. Alle Angaben in « Teilen » betreffen Gewichsteile. Alle

Angaben in « NCO-Äquivalent-% » beziehen sich auf die Gesamtmenge der in den Beispielen eingesetzten Ausgangsisocyanate.

## Beispiele

In den nachfolgenden Herstellungsbeispielen 1-37 wird als « Diisocyanat I » das, bei einem Druck von 2,3 mbar und innerhalb des Temperaturbereichs von 185 bis 203 °C destillierte, Diisocyanatgemisch gemäß Beispiel 1 der EP-A-58 368 eingesetzt.

« Katalysator A » ist ein Gemisch der Verbindungen

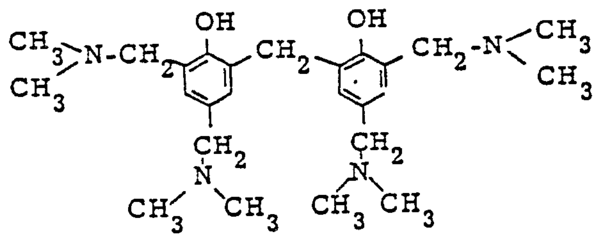

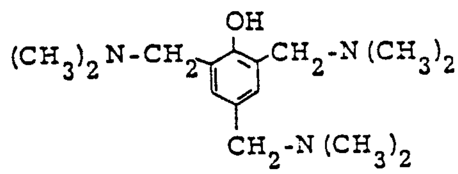

im Verhältnis 1 : 2,75.

« Katalysator B » besteht im wesentlichen aus der Mannich-Base.

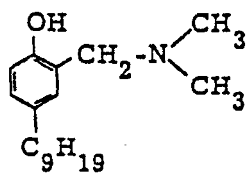

Die Herstellung dieser Katalysatoren erfolgte gemäß der Lehre der DE-A-2 551 634.

## Beispiel 1

In einem Dreihalskolben mit Rührer, Tropftrichter, Innenthermometer und Blasenzähler werden unter Stickstoff 800 Teile eines Gemischs aus 98 % 2,4-Toluylendiisocyanat und 2 % 2,6-Toluylendiisocyanat und 200 Teile Diisocyanat I (7,1 NCO-Äquivalent-%) in 1 000 Teilen einer Mischung von Xylol und Butylacetat (Gewichtsverhältnis 1 : 1) gelöst, unter Rühren auf 50 °C erwärmt und mit 0,4 Gew.-%, bezogen auf eingesetztes Isocyanat, des Katalysators (A) versetzt. Die langsam einsetzende Trimerisation wird 30 Stunden bei 50 °C aufrechterhalten. Danach ist der NCO-Gehalt auf 7,9 %gefallen. Die Reaktion wird durch Zugabe von 0,5 Gew.-%, bezogen auf eingesetztes Isocyanat, Toluolsulfonsäuremethylester gestoppt und das Reaktionsgemisch 1 h bei 50 °C nachgerührt. Man erhält als klare, farblose Lösung ein Isocyanuratgruppen aufweisendes Polyisocyanat-Gemisch mit den Daten : NCO-Gehalt : 7,9 % ; freies Toluylendiisocyanat : 0,15 % ; freies Diisocyanat I : 0,29 % : Viskosität (23 °C) : 1 100 mPas.

Entsprechend Beispiel 1 wurden die nachfolgend aufgelisteten Beispiele 2-9 durchgeführt (Tabelle I).

Die erhaltenen monomerenarmen, Isocyanurat-Gruppen enthaltenen Polyisocyanate der Beispiele 1-9 sind für die Herstellung von PUR-Lacken geeignet.

(Siehe Tabelle I Seite 6 f.)

Die folgenden Beispiele 10 und 11 beschreiben das erfindungsgemäße Verfahren bei der Herstellung von Isocyanuratgruppen enthaltenden Polyisocyanat-Gemischen, die für geschäumte Polyurethane geeignet sind.

## Beispiel 10

Zu einer Mischung von 500 Teilen Toluylendiisocyanat (80 % 2,4-, 20 % 2,6-Isomeres) und 500 Teilen Diisocyanat I (35,2 NCO-Äquivalent-%) werden bei 50 °C unter Rühren 0,1 Gew.-% des Katalysators (B) nach Beispiel 1 zugesetzt. Man läßt 5 Stunden bei 50 °C rühren, danach ist der NCO-Gehalt auf 28,7 % gefallen. Die Reaktion wird mit 0,1 Gew.-% Toluolsulfonsäuremethylester gestoppt und das Reaktionsgemisch 1 h bei 50 °C nachgerührt.

Man erhält eine klare Lösung des Isocyanuratgruppen aufweisenden Polyisocyanat-Gemischs mit den Daten : NCO-Gehalt : 28,7 % ; freies Toluylendiisocyanat : 26,8 %; freies Diisocyanat I : 30,2 % ; Viskosität (23 °C) : 1 100 mPas.

Tabelle I

| Beisp. | Einsatzmengen | | | | | Produktdaten | | | | |
|---|---|---|---|---|---|---|---|---|---|---|
| | Isocyanate | | | | Lösungsmittel | | | | | |
| | Toluylen-diisoc. 2,4- | 2,6-T. | Diisocyanat I (Teile) | Diisocyanat I NCO-Äquivalent-% | | Kat. A | Kat. B | % NCO | Toluylendiisoc./ Diisoc. (I) (% Restanteile) | Viskos. 23°C/mPa.s |
| 2 | 900 | - | 100 | 5,6 | Xylol/Butylacetat (1:1) | 0,4 | - | 8,6 | 0,26/0,17 | 2200 |
| 3 | 850 | - | 150 | 8,7 | " | 0,4 | - | 8,75 | 0,31/0,1 | 2200 |
| 4 | 800 | 75 | 125 | 7,1 | " | 0,4 | - | 8,2 | 0,13/0,18 | 1400 |
| 5 | 825 | 25 | 150 | 8,7 | " | 0,4 | - | 8,3 | 0,22/0,25 | 700 |
| 6 | 800 | 25 | 175 | 10,2 | " | 0,4 | - | 8,0 | 0,21/0,38 | 1000 |
| 7 | 720 | 180 | 100 | 5,6 | Butylacetat | 0,4 | - | 9,0 | 0,3/0,3 | 400 |
| 8 | 800 | - | 200 | 11,8 | Ethylacetat | 0,4 | - | 6,1 | 0,57/0,12 | 50 |
| 9 | 450 | 50 | 500 | 35,2 | Leichtbenzin/ Solvesso* 100 1:1 | 0,4 | 0,4 | 6,4 | 0,26/0,27 | 1600 |

Alle Produkte sind klare, farblose oder schwach gelb gefärbte Lösungen.

* ®Solvesso 100 ist ein handelsübliches Gemisch von Alkylaromaten mit einem über dem Siedepunkt von Xylol liegenden Siedebereich.

Beispiel 11

Beispiel 10 wird wiederholt mit dem Unterschied, daß 500 Teile Toluylendiisocyanat mit 0,1 Gew.-% Katalysator bis zu einem NCO-Gehalt von 34 % vortrimerisiert werden und nachfolgend 500 Teile Diisocyanat I innerhalb von 15 min zudosiert werden. Man läßt 30 Minuten bei 50 °C nachrühren, stoppt die Trimerisierungsreaktion und erhält ein Polyisocyanat-Gemisch mit den Daten : NCO-Gehalt : 28,8 % ; freies Toluylen-diisocyanat : 18,9 % ; freies Diisocyanat I : 40,3 % ; Viskosität (23 °C) : 1 200 mPas.

Beispiel 12

Entsprechend Beispiel 10 werden 500 Teile 4,4'-Diisocyanatodiphenylmethan und 500 Teile Diisocyanat I (entsprechend 38 NCO-Äquivalent-%) zur Reaktion grebracht. Man erhält ein Feststoff-freies Polyisocyanat-Gemisch mit den Daten : NCO-Gehalt : 23,7 %, Viskosität (23 °C) : 9 000 mPas.

Beispiel 13

Man verfährt wie in Beispiel 1 angegeben und trimerisiert 500 Teile des Diisocyanats I mit 0,8 Gew.-% Katalysator A und 0,1 Gew.-% Katalysator B bei 50-60 °C in 45 Stunden in 500 Teilen Testbenzin (Siedebereich 155-185 °C). Man erhält eine klare, farblose ca. 50 %ige Lösung mit einem NCO-Gehalt von 6,9 %. In der Lösung sind noch 3,2 % unverändertes Diisocyanat enthalten.

Versucht man im Vergleich hierzu ein Gemisch (80 : 20) von 2,4- und 2,6-Toluylendiisocyanat zu trimerisieren, tritt nach kurzer Zeit eine Trübung auf. Die Lösung wird inhomogen und bildet zwei Phasen.

Beispiel 14

150 Teile des Diisocyanats I und 850 Teile 2,4-Toluylendiisocyanat werden vermischt und mit 100 Teilen eines Hydroxyl-Polyesters aus Adipinsäure, Hexandiol-1,6 und Neopentylglykol mit dem Molgewicht ca. 1 700 bei 80 °C zur Reaktion gebracht, bis praktisch alle OH-Gruppen umgesetzt sind. Das so erhaltene Umsetzungsprodukt enthält 8,4 NCO-Äquivalent-% freies Diisocyanat I, 90,5 NCO-Äquivalent-% freies Toluylendiisocyanat und 1,1 NCO-Äquivalent-% aus Diisocyanat I, Toluylendiisocyanat und Hydroxyl-polyester gebildetes NCO-Prepolymer. Nun löst man in 1 100 Teilen eines Gemischs von Xylol/Butylacetat 1 : 1 auf und verfährt weiter wie in Beispiel 1 angegeben, indem man die Lösung bei 50 °C hält und 0,5 Gew.-% Katalysator A (bezogen auf eingesetztes Diisocyanat-Gemisch) zugibt. Nach 25 Stunden bei dieser Temperatur stoppt man mit 0,5 Gew.-% (ebenfalls bezogen auf das Diisocyanatgemisch) p-Toluolsulfonsäuremethylester ab.

Man erhält eine klare, praktisch farblose ca. 50 %ige Lösung mit einem NCO-Gehalt von 7,7 % und einer Viskosität von 900 mPas/23 °C. Der Restgehalt der Lösung an Diisocyanat-Gemisch beträgt 0,8 %.

Beispiel 15

In diesem Beispiel werden einige nach dem erfindungsgemäßen Verfahren hergestellte Polyisocyanate als Härter für einen 2-Komponenten-PUR-Holzlack eingesetzt.

Die Produkte werden mit einer Polyesterkomponente (Alkydharztyp) kombiniert. Dieses Alkydharz wird durch Schmelzkondensation von 30 Teilen Erdnußölfettsäure, 32 Teilen Triethylenglykol und 35 Teilen Phthalsäureanhydrid hergestellt. Es hat eine Hydroxylzahl von 150, eine Säurezahl von 8 und ist 75 %ig in Xylol gelöst.

Die nach dem erfindungsgemäßen Verfahren hergestellten Härter werden verglichen mit einem handelsüblichen Härter ($H_0$), gemäß Stand der Technik, der analog Beispiel 1 durch Trimerisierung von 2,4-Toluylendiisocyanat in 50 %iger Butylacetat-Lösung hergestellt wird. (Eine Herstellung in Xylol/Butylacetat im Gegensatz zum erfindungsgemäßen Verfahren ist nicht möglich, weil dann eine inhomogene, trübe Lösung entsteht). Die 50 %ige Lösung des Vergleichshärters hatte einen NCO-Gehalt von 7,7 % und eine Viskosität von 700 mPas/23 °C.

Um einen brauchbaren Vergleich zu erhalten, wurde ein Gew.-Verhältnis von Alkydharz : Härter wie 60 : 40 (fest/fest) gewählt. Dabei wird ein NCO/OH-Verhältnis von ungefähr 1 eingehalten. Alle Lacklösungen wurden mit Butylacetat auf 45 % Feststoff eingestellt.

Trocknungsgeschwindigkeit

Zur Prüfung der Trocknungsgeschwindigkeit wurden jeweils mit einer Lackhantel von 90 µm, 150 µm und 210 µm Filme auf Glas aufgezogen. Bestimmt wurden die Sandtrocknung und die Handtrocknung.

Das Ergebnis geht aus Tabelle II hervor :

Die Trocknung ist bei allen Lacken sehr schnell, ebenso schnell oder schneller wie mit dem handelsüblichen Härter $H_0$. Zwischen den einzelnen Produkten ist ein wesentlicher Unterschied nicht festzustellen.

Tabelle II

| Lackansatz m. Härter | | Sandtrocknung* (min.) | Handtrocknung* (min.) |
|---|---|---|---|
| $H_1$ (Beispiel 1) | 90 µm | 7 | 10 |
| | 150 µm | 10 | 14 |
| | 210 µm | 13 | 20 |
| $H_2$ (Beispiel 2) | 90 µm | 7 | 9 |
| | 150 µm | 11 | 13 |
| | 210 µm | 13 | 20 |
| $H_3$ (Beispiel 3) | 90 µm | 6 | 10 |
| | 150 µm | 10 | 15 |
| | 210 µm | 12 | 24 |
| $H_4$ (Beispiel 5) | 90 µm | 5 | 9 |
| | 150 µm | 9 | 13 |
| | 210 µm | 10 | 18 |
| $H_5$ (Beispiel 6) | 90 µm | 6 | 10 |
| | 150 µm | 9 | 14 |
| | 210 µm | 11 | 19 |
| $H_6$ (Beispiel 8) | 90 µm | 7 | 9 |
| | 150 µm | 10 | 13 |
| | 210 µm | 12 | 19 |
| $H_7$ (Beispiel 9) | 90 µm | 8 | 10 |
| | 150 µm | 10 | 14 |
| | 210 µm | 12 | 20 |
| $H_0$ (Vergleichs-produkt) | 90 µm | 7 | 10 |
| | 150 µm | 11 | 15 |
| | 210 µm | 14 | 23 |

* Sandtrocknung : Auf die lackierte Platte wird Sand gestreut. Man wartet 1 min. und versucht dann mit einem Pinsel den Sand zu entfernen. Die Zeit, nach der sich der Sand restlos entfernen läßt, wird als Trocknungszeit angegeben.

Handtrocknung : Man drückt mit dem Zeigefinger auf den Lackfilm. Der Zeitraum, nach dem kein Eindruck mehr entsteht, wird angegeben.

Viskositätsverlauf/Verarbeitungszeit

Der Viskositätsverlauf der Lackansätze wurde über die Auslaufzeit im DIN-4-Becher (DIN 53211) verfolgt (Tabelle III).

Tabelle III

| Lackansatz mit Härter | Auslaufzeit im DIN-4-Becher (sec.) | | | |
|---|---|---|---|---|
| | nach 0 h | nach 4 h | nach 6 h | nach 8 h |
| $H_1$ | 18 | 36 | 92 | x |
| $H_2$ | 18 | 40 | 88 | x |
| $H_3$ | 19 | 42 | x | x |
| $H_4$ | 18 | 30 | 70 | x |
| $H_5$ | 20 | 30 | 64 | 99 |
| $H_6$ | 18 | 33 | 42 | 90 |
| $H_7$ | 18 | 30 | 40 | 88 |
| $H_0$ | 27 | x | x | x |

x = Ansatz geliert

Die Viskosität der Lackansätze mit den Härtern, die nach dem erfindungsgemäßen Verfahren hergestellt, wurden, ist sofort nach dem Vermischen der Reaktionspartner niedriger als die Viskosität des Lackes mit dem Vergleichshärter. Der Grund liegt offensichtlich in der besseren Verträglichkeit dieser Härter mit den anderen Bestandteilen des Lackansatzes. Der Lackansatz mit dem Vergleichshärter $H_0$ war bereits nach 70 min. geliert. Die Lackansätze nach dem erfindungsgemäßen Verfahren lassen sich dagegen praktisch einen Arbeitstag lang einsetzen. Sie haben eine vorteilhaft lange Verarbeitungszeit.

Weiterhin wurde auch die Härte der Filme über die Pendeldämpfung (DIN 53 157) geprüft. Alle Lackfilme hatten schon nach einem Tag ebenso wie der Vergleichsversuch eine Pendeldämpfung von 120-140 s, nach 14 Tagen von 170-180 s.

Auch die Lösungsmittelbeständigkeit der Filme war nach 7 Tagen bei allen Filmen sehr gut.

Beispiel 16

Wichtige Bindemittel für die Holzlackierung sind Nitrocellulose und Celluloseacetobutyrat. Mit beiden Bindemitteln sind aromatische Isocyanuratgruppen enthaltende Isocyanate gemäß Stand der Technik unverträglich.

Die nach dem erfindungsgemäßen Verfahren hergestellten Härter $H_1$ bis $H_6$ werden mit Butylacetat auf 25 %ige Lösungen verdünnt und dann mit der 10 %igen Lösung Collodiumwolle-Chips in Butylacetat bzw. mit der 20 %igen Lösung eines Cellulosecetobutyrat-Typs (Cellit BP 300) im Verhältnis (fest auf fest) von 9 : 1, 8 : 2 und 7 : 3 vermischt. Alle Lösungen und die entsprechenden Filme sind klar. $H_0$ als Vergleichshärter bildet mit Celluloseacetobutyrat trübe Lösungen und einen unbrauchbaren, trüben Lackfilm, mit Nitrocellulose ist die Lösung ebenfalls leicht getrübt, der Film klar.

Beispiele 17-21 beschreiben die Ausführung des erfindungsgemäßen Verfahrens in einem Weichmacher. Auch in diesem Medium sind die Verfahrensprodukte überraschend gut löslich. Sie lassen sich zur Herstellung von lösungsmittelfreien Lacken einsetzen.

Beispiel 17

Zu einer Lösung von 250 Teilen Diisocyanat I in 705 Teilen Mesamol® (Lieferant : Bayer AG, Leverkusen ; PVC-Weichmacher auf Basis von Alkansulfonsäurearylester) werden bei 60 °C unter Rühren und Schutzgas ($N_2$) 1 Gew.-%, bezogen auf eingesetztes Diisocyanat, Katalysator (B) zugegeben. Man läßt bei 60 °C rühren, bis ein NCO-Wert von 3,2 % erreicht ist (ca. 4 bis 6 Stunden), gibt langsam 0,5 Gew.-%, bezogen auf eingesetztes Diisocyanat, p-Toluolsulfonsäuremethylester zu und läßt 1 Stunde bei 60 °C nachrühren. Man erhält eine klare Lösung mit einer Viskosität von 275 mPas (23 °C).

Entsprechend Beispiel 17 wurden die nachfolgend aufgelisteten Beispiele 18-21 durchgeführt (Tabelle IV).

Tabelle IV

| Beispiel | Reaktionstemp. | Reaktionszeit | Katalysator (B) (Teile) | NCO (%) | Viskosität (23°C/mPas) |
|---|---|---|---|---|---|
| 18 | 60°C | 70 min. | 1 | 3,6 | 275 |
| 19 | 60°C | 7 h | 1,1 | 3,0 | 290 |
| 19 | 60°C | 12 h | 1,3 | 2,8 | 320 |
| 21 | RT | 6 d | 1,4 | 2,5 | 400 |

Die Beispiele 22 bis 28 beschreiben die Ausführung des erfindungsgemäßen Verfahrens in Substanz (ohne Mitverwendung anderer Isocyanate). Die Verfahrensprodukte sind überraschenderweise nichtkristalline Flüssigkeiten. Sie lassen sich zur Herstellung von lösungsmittelfreien Beschichtungen einsetzen.

Beispiel 22

100 Teile Diisocyanat I werden mit 0,35 Teilen Katalysator B bei 60 °C unter Rühren und Schutzgas ($N_2$) zur Reaktion gebracht. Man läßt bei 60 °C rühren, bis ein NCO-Wert von 22,6 % erreicht ist (ca. 2 h), gibt langsam 0,35 Teile p-Toluolsulfonsäuremethylester zu und läßt 1 h bei 60 °C nachrühren. Man erhält eine klare Flüssigkeit mit einer Viskosität von 60 mPa.s (23 °C).

Entsprechend Beispiel 22 wurden die nachfolgend aufgelisteten Beispiele 23-28 durchgeführt (Tabelle V).

Tabelle V

| Beisp. | Reaktions-temperatur | Reaktions-zeit | Katalysator (B) (Teile) | NCO (%) | Viskosität (23°C/mPa.s) |
|---|---|---|---|---|---|
| 23 | 60°C | 3 h | 0,35 | 21,1 | 300 |
| 24 | 60°C | 5 h | 0,35 | 18,4 | 2000 |
| 25 | 70°C | 6 h | 0,35 | 16,1 | 22500 |
| 26 | RT | 2 Tage | 0,55 | 17,7 | 3700 |
| 27 | RT | 3 Tage | 0,55 | 15,6 | 38000 |
| 28 | 70°C | 8 h | 0,35 | 14,3 | 80000 |

Beispiel 29

672 g (4 Mol) Hexamethylendiisocyanat (HDI) und 260 g (0,8 Mol) Diisocyanat I (NCO-Gehalt : 43,3 %) werden unter Rühren und Feuchtigkeitsausschluß bei 50 °C mit 2 ml einer 0,5-molaren Lösung von Kaliumacetat in Polyethylenglykol (mittl. Molgewicht 370) zur Reaktion gebracht. Nach einer Reaktionszeit von 90 Minuten ist der Anfangsbrechungsindex : $n_D^{25}$ = 1,4696 auf $n_D^{25}$ = 1,4850 gestiegen. Durch Zugabe von 1 ml einer Lösung Perfluorbutensäure (10 Gew.-%) in HDI wird die Reaktion beendet. Man läßt 1 h bei 50 °C nachrühren und erhält ein Reaktionsprodukt mit einem NCO-Gehalt von 33,9 % (Trimerisierungsgrad : 21,7 %). Durch Dünnschichtdestillation gewinnt man 381 g eines hochviskosen, klarsichtigen Harzes mit den Daten : NCO-Gehalt : 12,4 %, freies HDI : 0,25 %, freies Diisocyanat I : < 0,1 %.

Der Anteil an eingebautem HDI im Trimerisat beträgt 33,3 %.

Beispiel 30

840 g (5 Mol) HDI und 162 g (0,5 Mol) Diisocyanat I (NCO-Gehalt : 45,6 %) werden bei 50 °C mit 4 ml einer Lösung des Katalysators N-Trimethyl-N-(2-hydroxypropyl)-ammoniumhydroxid (ca. 4 %ige Lösung in Ethylhexanol/Methanol = 8 : 1) zur Reaktion gebracht. Nach 2 h bei 50 °C ist der Anfangsbrechungsindex $n_D^{26}$ °C = 1,4618 auf $n_D^{26}$ °C = 1,4790 gestiegen. Das Reaktionsprodukt wird innerhalb von 15 Minuten auf 130 °C erhitzt und 30 Minuten bei dieser Temperatur nachgerührt. Danach sind Brechungsindex und NCO-Gehalt konstant : $n_D^{26}$ °C = 1,4845/NCO : 32,2 % (Trimerisierungsgrad 29,4 %). Nach Dünnschichtdestillation erhält man 490 g eines Isocyanuratgruppen aufweisenden Polyisocyanatgemisches als hochviskoses, klarsichtiges Harz mit den Daten : NCO-Gehalt : 15,5 %, freies HDI : 0,32 %, freies Diisocyanat I < 0,1 %.

Der Anteil an eingebautem HDI im Harz beträgt 67,7 %.

Beispiel 31

Gemäß Beispiel 30 werden 840 g (5 Mol) HDI und 81 g (0,25 Mol) Diisocyanat I (NCO-Gehalt : 47,5 %) mit 3,5 ml Katalysator zur Reaktion gebracht, bis ein NCO-Gehalt von 32,2 % (Trimerisierungsgrad : 32,2 %) erreicht ist. Nach Dünnschichtdestillation erhält man 494 g Produkt mit den Daten : NCO-Gehalt : 17,8 %, freies HDI : 0,51 %, freies Diisocyanat I : < 0,1 Gew.-%, Viskosität (23 °C) : ca. 60 000 mPa.s, eingebautes HDI : 84,1 %.

Beispiel 32

1 344 g (8 Mol) HDI (NCO-Gehalt : 50 %) werden unter Rühren und Feuchtigkeitsausschluß auf 50 °C erwärmt, mit 6 ml Katalysator gemäß Beispiel 30 versetzt und 1 h bei 50 °C gerührt. Danach ist der NCO-Gehalt auf 48,5 % (Trimerisierungsgrad 3 %) gefallen. Anschließend läßt man innerhalb von 40 Minuten bei 50 °C 260 g (0,8 Mol) Diisocyanat I zutropfen. Nach einer Reaktionszeit von 80 Minuten wird das Reaktionsprodukt innerhalb von 15 Minuten auf 130 °C erhitzt und 30 Minuten bei dieser Temperatur nachgerührt. Danach sind Brechungsindex und NCO-Wert konstant : $n_D^{24}$ = 1,4808/NCO : 33,0 % (Trimerisierungsgrad : 27,6 %). Durch Dünnschichtdestillation gewinnt man 698 g eines Isocyanuratgruppen aufweisenden Polyisocyanatgemischs als hochviskoses, klarsichtiges Harz mit den Daten : NCO-Gehalt : 15,5 %, freies HDI : 0,37 %, freies Isocyanat der Formel (I) < 0,1 %, eingebautes HDI : 63,1 %.

Beispiel 33

1 344 g (8 Mol) HDI werden gemäß Beispiel 32 mit 6 ml Katalysator zur Reaktion gebracht, bis der NCO-Gehalt 48,7 % beträgt (Trimerisierungsgrad 2,6 %). Danach werden innerhalb von 30 Minuten 130 g (0,4 Mol) Diisocyanat I eingetropht. Man verfährt gemäß Beispiel 33, bis ein NCO-Gehalt von 34,5 % erreicht ist (Trimerisierungsgrad : 25,3 %). Durch Dünnschichtdestillation erhält man 713 g Produkt mit den Daten : NCO-Gehalt : 18,0 %, freies HDI : 0,55 %, freies Diisocyanat der Formel (I) < 0,1 %, Viskosität (23 °C) : ca. 55 000 mPa.s, eingebautes HDI : 82,1 %.

Beispiel 34

1 332 g (6 Mol) 3,5,5-Trimethyl-1-isocyanato-3-isocyanatomethylcyclohexan (Isophorondiisocyanat) und 97 g (0,3 Mol) Diisocyanat I (NCO-Gehalt 37,0 %) werden bei 60-70 °C innerhalb von 1 h mit 16 ml Katalysator gemäß Beispiel 30 versetzt. Man läßt 3 h bei 70 °C nachrühren, erhitzt anschließend das Reaktionsprodukt innerhalb von 15 Minuten auf 130 °C, und rührt 30 Minuten bei dieser Temperatur. Danach sind Brechungsindex und NCO-Gehalt konstant : $n_D^{24}$ = 1,4960/NCO : 30,2 % (Trimerisierungsgrad : 18,4 %). Nach Dünnschichtdestillation erhält man 262 g eines Isocyanuratgruppen aufweisenden Polyisocyanats mit den Daten : NCO-Gehalt : 13,8 %, freies Isophorondiisocyanat : 0,50 %, freies Isocyanat der Formel (I) < 0,1 %, eingebautes Isophorondiisocyanat : 78,1 %.

Aussehen : sprödes, klares Harz.

Beispiel 35

Entsprechend Beispiel 1 werden 900 Teile Toluylendiisocyanat und 100 Teile Diisocyanat I (5,6 NCO-Äquivalent-%) in Xylol/Butylacetat gelöst und bei Raumtemperatur mit 0,2 Gew.-% Katalysator B versetzt. Die langsam einsetzende Trimerisation wird 22 h bei Raumtemperatur aufrechterhalten. Danach ist der NCO-Gehalt auf 8,4 % gefallen. Man stoppt durch Zugabe von 0,2 Gew-% Toluolsulfonsäuremethylester und erhält eine klare Lösung mit den Daten : NCO-Gehalt : 8,4 %, freies Toluylendiisocyanat : 0,24 %, freies Diisocyanat : I 0,1 %, Viskosität (23 °C) : 2 100 mPa.s.

Beispiel 36

Beispiel 35 wird wiederholt unter Verwendung des Diisocyanats gemäß Beispiel 6 der US-A 2 986 576 mit verzweigter Alkylkette. Nach 30 h Reaktion bei Raumtemperatur und nachfolgendem Abstoppen erhält man eine klare Lösung mit den Daten : NCO-Gehalt : 7,9 %, freies Toluylendiisocyanat : 0,44 %, freies Diisocyanat gemäß US-A 2 986 576 : 0,36 %, Viskosität (23 °C) : 4 700 mPa.s.

Beispiel 37

Entsprechend Beispiel 36 wird unter Mitverwendung von 0,3 Gew.-% Katalysator B nach 9 h Reaktion bei Raumtemperatur und Abstoppen mit 0,3 Gew.-% Toluolsulfonsäuremethylester eine klare Lösung erhalten mit den Daten : NCO-Gehalt : 8,5 %, freies Toluylendiisocyanat : 0,51 %, freies Diisocyanat gemäß US-A 2 986 576 : > 0,1 %, Viskosität (23 °C) 2 400 mPa.s.

**Patentansprüche**

1. Verfahren zur Herstellung von Isocyanurat-Gruppen aufweisenden Polyisocyanaten durch Trimerisierung eines Teils der Isocyanatgruppen von organischen Polyisocyanaten in Gegenwart von Trimerisierungskatalysatoren und Abbruch der Trimerisierungsreaktion beim jeweils gewünschten Trimerisierungsgrad durch Zugabe eines Katalysatorengifts und/oder durch thermische Zersetzung des eingesetzten Trimerisierungskatalysators, dadurch gekennzeichnet, daß man als zu trimerisierende Polyisocyanate

a) Verbindungen oder Gemische von Verbindungen der Formel

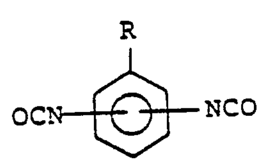

in welcher R für einen gesättigten, aliphatischen Kohlenwasserstoffrest mit 8 bis 15 Kohlenstoffatomen steht, und gegebenenfalls

b) bis zu 95 NCO-Äquivalent-%, bezogen auf die Summe a) + b), an anderen organischen

Polyisocyanaten verwendet.

2. Ausführungsform des Verfahren gemäß Anspruch 1, dadurch gekennzeichnet, daß man in einem ersten Reaktionsschritt bis maximal 30 % der Isocyanatgruppen der Ausgangspolyisocyanate a) oder b) trimerisiert, anschließend die zweite Ausgangskomponente b) oder a) dem Reaktionsgemisch hinzufügt und die Reaktion zu Ende führt, wobei der Abbruch der Trimerisierungsreaktion frühestens dann erfolgt, wenn mindestens weitere 5 % der Isocyanatgruppen, bezogen auf alle Isocyanatgruppen der untrimerisierten Ausgangspolyisocyanate, trimerisiert sind.

3. Verfahren gemäß Anspruch 1 und 2, dadurch gekennzeichnet, daß man als Verbindungen a) Gemische von Homologen und Isomeren von Diisocyanaten der in Anspruch 1 genannten Formel verwendet, in welcher R für einen geradkettigen Rest steht.

4. Verfahren gemäß Anspruch 1 bis 3, dadurch gekennzeichnet, daß man als Ausgangspolyisocyanat b) 2,4- und/oder 2-6-Diisocyanatotoluol verwendet.

5. Verfahren gemäß Anspruch 1 bis 4, dadurch gekennzeichnet, daß man als Ausgangspolyisocyanate, bezogen auf die in den nicht-trimerisierten Ausgangspolyisocyanaten vorliegenden Isocyanatgruppen,

a) 5 bis 70 Äquivalent % an Homologen und Isomeren von Diisocyanaten der in Anspruch 1 genannten Formel, wobei R für einen geradkettigen Rest steht, und

b) 30 bis 95 NCO-Äquivalent-% an 2,4-Diisocyanatotoluol oder dessen technischen Gemischen mit bis zu 65 Gew.-%, bezogen auf die Gesamtmenge der Diisocyanatotoluol-Isomeren, an 2,6-Diisocyanatotoluol

verwendet.

6. Gemäß Anspruch 1 bis 5 erhältliche, Isocyanurat-Gruppen aufweisende Polyisocyanate.

7. Verwendung der gemäß Anspruch 1 bis 5 erhältlichen, Isocyanurat-Gruppen aufweisenden Polyisocyanate, gegebenenfalls in von überschüssigen Ausgangspolyisocyanaten befreiter Form und/oder gegebenenfalls in mit Blockierungsmitteln für Isocyanat-Gruppen blockierter Form als Isocyanatkomponente zur Herstellung von Polyurethanen nach dem Isocyanat-Polyadditions-Verfahren.

**Claims**

1. A process for the production of polyisocyanates containing isocyanurate groups by trimerization of some of the isocyanate groups of organic polyisocyanates in the presence of trimerization catalysts and termination of the trimerization reaction at the particular degree of trimerization required by addition of a catalyst poison and/or by thermal decomposition of the trimerization catalyst used, characterized in that

a) compounds or mixtures of compounds corresponding to the following formula

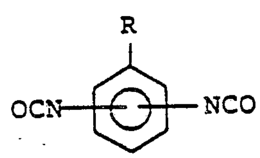

in which R is a saturated, aliphatic $C_8$-$C_{15}$ hydrocarbon radical and, optionally,

b) up to 95 NCO-equivalent-%, based on the sum of a) + b), of other organic polyisocyanates are used as the polyisocyanates to be trimerized.

2. An embodiment of the process claimed in claim 1, characterized in that, in a first reaction step, up to at most 30 % of the isocyanate groups of the starting polyisocyanates a) or b) are trimerized, after which the second starting component b) or a) is added to the reaction mixture and the reaction is completed, the trimerization reaction being terminated at the earliest when at least another 5 % of the isocyanate groups, based on all the isocyanate groups of the untrimerized starting polyisocyanates, have been trimerized.

3. A process as claimed in claims 1 and 2, characterized in that mixtures of homologs and isomers of diisocyanates corresponding to the formula in claim 1, in which R is a straight-chain radical, are used as the compounds a).

4. A process as claimed in claims 1 to 3, characterized in that 2,4- and/or 2,6-diisocyanatotoluene is/are used as the starting polyisocyanate.

5. A process as claimed in claims 1 to 4, characterized in that

a) 5 to 70 equivalent-% of homologs and isomers of diisocyanates corresponding to the formula in claim 1, in which R is a straight-chain radical, and

b) 30 to 95 NCO-equivalent-% of 2,4-diisocyanatotoluene or technical mixtures thereof with up to 65 % by weight, based on the total quantity of the diisocyanatotoluene isomers, of 2,6-diisocyanatotoluene,

based on the isocyanate groups in the non-trimerized starting polyisocyanates, are used as the starting polyisocyanates.

6. Polyisocyanates containing isocyanurate groups obtainable by the process claimed in claims 1 to 5.

7. The use of the polyisocyanates containing isocyanurate groups obtainable by the process claimed in claims 1 to 5, optionally freed from excess starting polyisocyanates and/or blocked with blocking agents for isocyanate groups, as isocyanate component for the production of polyurethanes by the isocyanate polyaddition process.

**Revendications**

1. Procédé de préparation de polyisocyanates portant des groupes isocyanurate par trimérisation d'une partie des groupes isocyanate de polyisocyanates organiques en présence de catalyseurs de trimérisation et interruption de la réaction de trimérisation au taux de trimérisation voulu dans chaque cas par addition d'un poison de catalyseur et/ou par décomposition à la chaleur du catalyseur de trimérisation mis en œuvre, caractérisé en ce que l'on utilise en tant que polyisocyanates à trimériser

a) des composés ou mélange des composés de formule

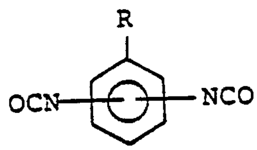

dans laquelle R représente un radical hydrocarboné aliphatique saturé en $C_8$-$C_{15}$, et le cas échéant

b) jusqu'à 95 équivalents % de NCO, par rapport à la somme a + b, d'autres polyisocyanates organiques.

2. Mode de réalisation du procédé selon la revendication 1, caractérisé en ce que, dans un premier stade de réaction, on trimérise jusqu'à un maximum de 30 % des groupes isocyanate des polyisocyanates de départ a) ou b), on ajoute ensuite au mélange de réaction le deuxième composant de départ b) ou a) et on termine la réaction, en interrompant la réaction de trimérisation au plus tôt lorsqu'on a trimérisé au moins encore 5 % des groupes isocyanate, par rapport à la totalité des groupes isocyanate des polyisocyanates de départ non trimérisés.

3. Procédé selon les revendications 1 et 2, caractérisé en ce que l'on utilise en tant que composés a) des mélanges d'homologues et d'isomères de diisocyanates de formule donnée dans la revendication 1 dans laquelle R représente un radical à chaîne droite.

4. Procédé selon les revendications 1 à 3, caractérisé en ce que l'on utilise en tant que polyisocyanate de départ b) le 2,4- et/ou le 2-6-diisocyanatotoluène.

5. Procédé selon les revendications 1 à 4, caractérisé en ce que l'on utilise en tant que polyisocyanates de départ, par rapport aux groupes isocyanate présents dans les polyisocyanates de départ non trimérisés,

a) 5 à 70 équivalents % d'homologues et d'isomères de diisocyanates de formule de la revendication 1, dans laquelle R représente un radical à chaîne droite, et

b) 30 à 95 équivalents de NCO % de 2,4-diisocyanatotoluène ou de ses mélanges techniques avec une proportion allant jusqu'à 65 % en poids, par rapport à la quantité totale des isomères de diisocyanatotoluène, de 2,6-diisocyanatotoluène.

6. Polyisocyanates portant des groupes isocyanurate, obtenus selon les revendications 1 à 5.

7. Utilisation des polyisocyanates portant des groupes isocyanurate obtenus selon les revendications 1 à 5, éventuellement débarrassés de l'excès des polyisocyanates de départ et/ou éventuellement à l'état bloqué par des agents bloquants des groupes isocyanate, en tant que composants isocyanates pour la préparation de polyuréthannes par le procédé de polyaddition des isocyanates.